Europäisches Patentamt

(19) European Patent Office

Office européen des brevets

(11) Publication number: **0 106 285**
**A2**

# (12) EUROPEAN PATENT APPLICATION

(21) Application number: **83110040.9**

(22) Date of filing: **07.10.83**

(51) Int. Cl.³: **A 61 K 39/00**
**A 61 K 37/02**

(30) Priority: **08.10.82 JP 177411/82**
**06.06.83 JP 100528/83**

(43) Date of publication of application:
**25.04.84 Bulletin 84/17**

(84) Designated Contracting States:
**AT BE CH DE FR GB IT LI NL SE**

(71) Applicant: **OTSUKA PHARMACEUTICAL CO., LTD.**
**9, Kandatsukasacho 2-chome**
**Chiyoda-ku Tokyo 101(JP)**

(72) Inventor: **Adachi, Masakazu**
**No. 3493-9, Ishihara-cho**
**Takasaki-shi Gunma(JP)**

(74) Representative: **Hansen, Bernd, Dr.rer.nat. et al,**
**Hoffmann, Eitle & Partner Patentanwälte Arabellastrasse**
**4**
**D-8000 München 81(DE)**

(54) Glycosidic linkage related antigen, process for producing the same and anticancer agent containing the same as effective component.

(57) A thermally denatured glycosidic linkage related antigen derived from cancerous cells is disclosed, which comprises a lectin receptor collected from cancerous cell membrane which can bind with a terminal galactose-and/or termainal N-acetylgalactosamine-binding lectin, and/or a cancerous cell membrane component having a property of binding with an antibody capable of binding with the lectin receptor, each of the lectin receptor and the cancerous cell membrane component being thermally denatured, the thermally denatured glycosidic likage related antigen containing a thermally denatured protein moiety and a thermally non-denatured saccharide moiety.

Further, a process for preparing a thermally denatrued glycosidic linkage related antigen derived from cancerous cells is disclosed.

Also, a cancerous cell membrane component having a property of binding with an antibody capable of binding with the lectin receptor, and a process for preparing the same as well as an anticancer agent comprising a thermally denatured glycosidic linkage related antigen derived from cancerous cells are disclosed.

EP 0 106 285 A2

GLYCOSIDIC LINKAGE RELATED ANTIGEN,
PROCESS FOR PRODUCING THE SAME AND
ANTICANCER AGENT CONTAINING THE SAME AS EFFECTIVE COMPONENT

The present invention relates to a novel glycosidic linkage related antigen derived from cancer cells (hereinafter referred to as "GRA") and, in greater detail, to GRA which specifically acts on cancerous cells having GRA to easily induce cancerous cell-cytotoxic lymphocytes which destroy the cancerous cells (hereinafter referred to as "killer cells").

It is known that immune response effector cells, particularly T lymphocytes playing a main role in cell-mediated immune response, cause rejection of grafts due to foreign cell antigens, but exhibit no appreciable or very limited immuno-inhibition against cancer cells. Thus, the cancer cells are not destroyed and multiply _in vivo_, finally putting the cancer-bearing host to death.

Previous research on the immune response of the host to cancer cells and its application to the treatment of cancer, has shown that, in a cancer cell specific antigen which is not found in differentiated normal cells, there is GRA which acts as an immunogen for the host and have very high immunogenicity that cause an immune response specific to the cancer cells and that when GRA is used to sensitize lymphocytes, there can be obtained killer cells which act specifically on cancer cells containing GRA and if the killer cells are administered to the host, they recognize GRA and act on the cancer cells containing GRA, destroying them, and thus that they exhibit an excellent effect in the treatment and prevention of cancer.

The above described GRA, which is a cancerous cell membrane component of GRA-containing cancerous cells, is a component which can bind a lectin capable of binding terminal galactose and/or terminal N-acetylgalactosamine (hereinafter referred to as "Lectin receptor").

In the subsequent state of studies, it has been found that cancerous cell membrane components contain a component having a property of binding an antibody which in turn is capable of binding the above described lectin receptor (hereinafter referred to as "GRA antibody"), the component being hereinafter referred to as "A-GRA". It has also been found that this A-GRA is novel GRA which has effects similar to those in the above described lectin receptor.

On the other hand, it has been known that, since the immune reaction to cancers (tumor rejection) is based chiefly on cell mediated immunity, while humoral immunity, which is caused by an antibody to a cancer associated antigen by masking the antigen, and, by acting as an inhibitory factor for the immune reaction (blocking antibody) by itself or by forming an immune complex, or by changing the distribution of the antigen (antigenic modulation), sometimes results in not only the destruction of the immunological surveillance mechanism of the living body but also the acceleration of the growth of tumors to bring a disadvantageous action for the host.

Extensive research has been made in order to obtain cancerous cell associated antigens capable of bringing about strong cell mediated immunity which is specific to cancerous cells and which prevents the occurrence of humoral immunity of the cancer carrying hosts (low antibody productivity), and as a result thereof it has been found that a thermally denatured antigen obtained by heat treatment of GRA composed of the above described lectin receptor and/or A-GRA (hereinafter referred to as "thermally denatured GRA") satisfies the above described requirements and the thermally denatured GRA can be used for medical treatment and prevention for cancers in a wide dosage range since it has a low dependence on the concentration.

The present invention is based on the above findings and provides the above-described thermally denatured GRA and a process for producing the same.

Further, the present invention provides an anticancer agent comprising the above described A-GRA or the thermally denatured GRA as an effective component.

<u>DETAILED DESCRIPTION OF THE INVENTION</u>

The A-GRA of the present invention can be obtained from GRA-containing cancerous cells such as cultured human or animal cancerous cells, transplanted cancerous cells, spontaneous cancerous cells, chemically or virally induced cancerous cells or cancerous cells originated in operation tissue, etc. as follows. Namely, a cell membrane component is firstly separated from the above described cancerous cells and thereafter treated with a GRA antibody, to bind A-GRA and isolate the A-GRA-GRA antibody complex. Further, the A-GRA can be separated by treating with a lectin capable of binding terminal galactose and/or N-acetylgalactosamine to bind it to the lectin.

Examples of the lectin which can specifically bind terminal galactose include peanut lectin (PNA), castor bean (<u>Ricinus Communis</u>) lectin and soybean lectin (SBA), etc. (<u>J.B.C.</u> 250, 8518-8523 (1975); <u>Biochem. Biophys. Res. Comm.</u> <u>62</u>, 144 (1975); <u>Z. Immunitaetsforch.</u> <u>138</u>, 423-433 (1969); <u>Br. J. Exp. Pathol</u>, <u>27</u>, 228-236 (1946); <u>Proc. Nat. Acad. Sci. U.S.A.</u>, <u>75</u>, No. 5, 2215-2219 (1978); <u>Biochemistry</u> 13, 196-204 (1974); and <u>Carbohydrate Research</u>, <u>51</u>, 107-118 (1976).

Examples of the lectin which can specifically bind terminal N-acetylgalactosamine include <u>Dolichos</u> bean lectin (DBA), braid orange lectin, <u>Hilix</u> <u>pomatia</u> lectin, lima bean lectin, SBA, <u>Bauhinia</u> bean lectin, etc.

As the GRA antibody, any material can be used, if it has a property of binding the lectin receptor. For example, it is possible to use autoantibodies of the patient with cancers, antisera of animals obtained by immunizing the animals with a lectin receptor as an antigen in a conventional manner, and monoclonal antibodies obtained by a known method (for example, the method described in <u>Nature</u>, <u>256</u>, 495-497 (1975)) from spleen cells immunized with lectin receptor-positive cancerous cells, or cell membrane components thereof or a lectin receptor as an antigen.

The autoantibodies of patients with cancers can be obtained as an antiserum which is collected according to the conventional method from GRA antibody-positive patients and can be used as is or as an immunoglobulin fraction obtained by purifying it as by salting-out, etc.

As the lectin receptor, it is possible to use those which are obtained by separating a component having a property of binding a lectin which is capable of binding terminal galactose and/or terminal N-acetylgalactosamine from the cancerous cell membrane component according to the process for producing A-GRA of the present invention. Mammals to be subjected to immunization are not especially limited. Generally, rabbits, mice, rats and the like are used, and the immunization can be carried out · by a conventional method. For example, the above described lectin receptor is diluted with physiological saline, etc. so as to have a suitable concentration and it is administered into the animal by intradermal injection as a suspension with a Ferund's adjuvant. It is preferred that the administration be carried out several times every 1 to 2 weeks so that the whole dosage becomes 1 µg to 20 mg or so in case of, for example, rabbits, and the GRA antibody is collected as a serum.

As the monoclonal antibody, it is possible to use those which are obtained by a method comprising screening a hybridoma cell line producing the desired antibody from hybridomas obtained by fusing spleen cells of an animal immunized as described above with known myeloma cells such as NS-1, P3, P3-U1, MPC-11, SP2, X63.6.5.3, etc., and separating as a supernatant of cultured medium in which the above-described hybridoma cells have been cultured, or multiplying the hybridoma cells by administering them into an animal which has compatibility therewith and separating from ascites.

Separation of the cancerous cell membrane component can be carried out by known methods, such as a homogenizing method, or a solubilizing method using a solubilizing agent. Preferably, it can be carried out by a method which comprises homogenizing cancerous cells in physiological saline or in a suitable buffer solution, separating precipitate by centrifugal separation, etc., dissolving it in physiological saline or in a buffer solution in the presence of a solubilizing agent, and separating the supernatant by centrifugal separation, etc.

As the solubilizing agent used, there are various surface active agents which are known to solubilize cell membranes, for example, nonionic surface active agents such as "Triton X-100" (produced by Wako Pure Chemical Industries, Ltd.), "NP-40" (produced by Shell Co.), digitonin or urea, etc. and anionic surface active agents such as sodium dodecylsulfate (SDS), etc.

Separation of the A-GRA which can bind GRA antibody from the cell membrane component can be carried out by conventional physicochemical or biochemical means utilizing properties of the A-GRA. Examples of such means include affinity chromatography utilizing a column carrier containing the above-described antibody, an immuno-precipitation process, a gel filtration process, an electrophoretic process, a physical precipitation process utilizing a glycoprotein precipitant such as polyethylene glycol or acetone, etc. and processes of suitable combinations of them.

Preferably, it is preferred to use affinity chromatography utilizing a column carrier containing the antibody. The column carrier can be easily obtained by fixing the above antibody on an insoluble support. The fixation of the antibody on the insoluble support can be carried out by the known methods of fixing the living matters. Among them, it is preferred to use fixation by a method using a polysaccharide activated with cyanogen bromide and a method using N-hydroxysuccinimide ester. The method using a polysaccharide activated with cyanogen bromide comprises treating an insoluble support with cyanogen bromide and coupling the resulted activated material with a GRA antibody under mild conditions to fix the GRA antibody to the support. In carrying out treatment of the insoluble support with cyanogen bromide, the support may be treated in water or acetonitrile at room temperature with keeping the pH at 7.5 to 12 with a basic compound such as sodium hydroxide or sodium hydrogen carbonate, etc. or in a buffer solution having a pH of 7.5 to 12 such as a 0.1M sodium hydrogen carbonate buffer solution having a pH of about 8.7 or a 0.01M phosphoric acid buffer solution having a pH of about 7.7, etc. for about 1 to 12 minutes. The amount of the cyanogen bromide used is generally nearly the same weight as that of the insoluble support. As the insoluble support, it is possible to use any known insoluble supports that show a low non-specific adsorption to living matters in general and have a high porosity, which have a functional group capable of fixing the living matters under mild conditions and are sufficiently stabilized chemically and physically. Examples of the insoluble support include cellulose supports such as aminoethyl cellulose, carboxymethyl cellulose, bromoacetyl cellulose or p-anilino cellulose, etc., cross-linked dextran supports such as Sephadex or CM-Sephadex (produced by Pharmacia Co.), etc. and agarose supports such as Sepharose 2B, Sepharose 4B or Sepharose 6B (produced by Pharmacia Co.), etc. When carrying out coupling of the resulted support activated with cyanogen bromide with the GRA antibody, the support activated with cyanogen bromide is used in an amount of 30 to 80 times by weight that of the GRA antibody, and the reaction is carried out generally at 0 to 40°C, preferably 2 to 8°C for about 10 to 20 hours in a suitable solvent, for example, a 0.1M aqueous solution of sodium hydrogen carbonate (containing 0.5M of sodium chloride, pH 8.4). Thus, the carrier for affinity chromatography can be produced.

According to the chromatography utilizing the above described carrier for affinity chromatography containing GRA antibody, the desired A-GRA can be caught on the column by being bound to the GRA antibody in the above described carrier. Then, the A-GRA is obtained by carrying out an exchange reaction by passing a substance capable of binding the GRA antibody through the column or by passing an adsorptive separator (eluting solution) such as a salt solution having a high concentration, an aqueous solution of potassium thiocyanate, a boric acid buffer solution or a hydrochloric acid-glycine buffer solution (pH=2.7), etc. through the column to separate A-GRA.

As the substances capable of binding GRA antibody used in the above described exchange reaction, there are substances which bind to lectin capable of binding to galactose, such as galactose, disacchrides having galactose in the terminal or oligosaccharides having galactose in the terminal, etc. and substances which bind to lectin capable of binding to N-acetylgalactosamine, such as N-acetylgalactosamine, disaccharides having N-acetylgalactosamine in the terminal or oligosaccharides having N-acetylgalactosamine in the terminal.

The resulting A-GRA of the present invention can be separated by treating with lectin capable of binding to terminal galactose and/or terminal N-acetylgalactosamine to bind to said lectin. The treatment with lectin can be carried out according to the above described affinity chromatography utilizing the column carrier containing lectin. As the column carrier, there are those available in the market and those obtained by the same method as the above described method of fixing the GRA antibody on the insoluble support. Further, chromatography utilizing a carrier for affinity chromatography containing the lectin can be carried out similarly to the above described affinity chromatography of the GRA antibody.

The A-GRA allowed to adsorb on the carrier is dissociated by an exchange reaction or with an adsorptive separator. The exchange reaction is carried out using the above described substances which can bind to a lectin capable of binding galactose when using the lectin capable of binding galactose as a carrier, or carried out using the above described substances which can bind a lectin capable of binding N-acetylgalactosamine when using the lectin capable of binding N-acetylgalactosamine as a carrier. In production of the above described A-GRA, a component which can bind a lectin (lectin receptor) is obtained from cancerous cell membranes by the above described treatment with lectin and, thereafter, subjected to the above described treatment with GRA antibody, by which the desired A-GRA can be obtained.

The A-GRA of the present invention obtained as described above contains glycoproteins, glycolipids and/or saccharides each having galactose and/or N-acetylgalactosamine in the terminal. This A-GRA, if necessary, can be purified or lyophilized by a conventional method.

The lectin receptor can be obtained by separating the above described component having a property of being bound to a lectin capable of binding terminal galactose and/ or terminal N-acetylgalactosamine from the above described cancerous cell membrane component according to the above described process for producing A-GRA, preferably by affinity chromatography utilizing the above described column carrier containing such lectin. This lectin receptor contains glycoproteins, glycolipids and/or saccharides each having galactose and/or N-acetylgalactosamine in the terminal, which can be, if necessary, lyophilized or further purified by a conventional separating means. For example, there is a process which comprises separating the lectin receptor separated with a lectin capable of binding galactose with a lectin capable of binding N-acetylgalactosamine or a process which comprises separating the lectin receptor separated with a lectin capable of binding N-acetylgalactosamine with a lectin capable of binding galactose.

Further, in the GRA antibody of the present invention, it has been found for the first time that the above-described antibody is present in the body fluid of patients with cancers as an autoantibody. This antibody is confirmed (measured) by the method described in the following and is collected from antibody-positive patients as described above.

Further, the present invention provides a method of measurement for the above described GRA antibody. The method of measurement is useful for diagnosis of cancer, for medical treatment resistance, and for judgment of prognosis.

The above-described method of measurement can be carried out by observing an antibody which can be bound to lectin receptor-positive cancerous cells by an indirect rosette method using the lectin receptor-positive cancerous cells, preferably, the same cancerous cells which do not express major histocompatibility complex (M.H.C.). Namely, the above described cancerous cells are suspended in a medium which is used for conventional cultivation of cells, such as RPMI-1640 medium, etc. so as to have a suitable population generally $3 \times 10^6$ cells/ml medium, and serum of a patient with cancer is added to the resulting cell suspension and a reaction is carried out at 4 to 37°C for 30 minutes to 4 hours. After the cancerous cells are sufficiently washed with the above described medium, red blood cells such as bovine red blood cells (ORBC), etc., protein A bound to polystyrene beads or latex, or anti-human immunoglobulin antibody bound to polystyrene beads or latex are added thereto and reacted at 4 to 37°C for several minutes to 30 minutes by centrifugation (about 1000 rpm). The cells are fixed and dyed by a conventional method and rosette forming cells are examined by visual observation, by which existence of GRA antibody in the serum of the patient can be confirmed. Namely, when using a serum of GRA antibody-positive patients, rosette formation is observed in the cancerous cells an the rate of formation is evaluated as a GRA antibody value.

As the GRA antibody used in the above described process for producing A-GRA, that collected from the serum of a patient having a high antibody value is preferred.

Thermal denaturation of the resulting GRA composed of A-GRA and/or the lectin receptor is carried out under a conventional condition for denaturing proteins. For example, it is carried out by heating GRA in a solvent such as water, physiological saline or a phosphoric acid buffer solution at about 60 to 120°C, preferably about 90 to 110°C for 5 to 60 minutes, preferably 10 to 20 minutes.

The thermally denatured GRA of the present invention is a glycoprotein composed of a saccharide terminal structure of galactose and/or N-acetylgalactosamine and a thermally denatured protein.

When lymphocytes are sensitized with the A-GRA or the thermally denatured GRA of the present invention, killer cells are produced.

The lymphocytes used here are not especially restricted, and any lymphocytes of normal or cancer-carrying humans or animals can be used. Examples of them include those derived from peripheral blood, bone marrow, lymph node, spleen, tonsil and thymus gland, etc. These lymphocytes can be isolated by, for example, a physical or chemical process or a surface membrane process, and they can be used for the process for producing killer cells.

Sensitization of lymphocytes with A-GRA can be carried out by cultivating the lymphocytes in a medium containing A-GRA for 1 to 3 days, preferably 2 days.

Sensitization of lymphocytes with thermally denatured GRA can be similarly carried out by incubating them for several hours to 10 days, preferably 1 to 5 days.

As the medium, various kinds of medium conventionally used for incubating this kind of cells can be used, but it is preferred to use, for example, RPMI 1640 medium and Eagle MEM medium to which human serum, fetus calf serum (FCS), calf serum or horse serum, etc. is added. The A-GRA or the thermally denatured GRA to be added to the medium is preferred to be in an amount of 0.1 to 1000 ng/ml, preferably 1 to 500 ng/ml as a protein based on the $1 \times 10^6$ lymphocytes/ml, when using A-GRA; and 1 to 2000 ng/ml, preferably 50 to 500 ng/ml as saccharide, when using thermally denatured GRA.

Incubation is carried out, for example, at a temperature of 37°C and at a pH of 7.2 or so according to the conventional method.

The thus obtained killer cells can be multiplied without any restriction in the above described medium containing T-cell growth factor (TCGF IL-2). In this case, selective incubation for cloning killer cells may be carried out by a conventional limiting dilution method. The killer cells can be stably preserved for a long period of time, if they are preserved in, for example, liquid nitrogen.

The resulting killer cells are substantially normal lymphocytes, which are characterized by having a cytotoxicity specific to GRA. These killer cells are held in a state available from the applicant.

The A-GRA and the thermally denatured GRA of the present invention obtained as described above are useful as an anticancer agent. The A-GRA and the thermally denatured GRA may be used alone as an effective ingredient. They may be used together with other antimicrobial agents and/or anticancer agents. The anticancer agent containing A-GRA or thermally denatured GRA of the present invention as an effective ingredient may have any form, if it is in a state of effectively containing A-GRA or thermally denatured GRA as a major active ingredient. However, it is generally administered by intravenous injection, subcutaneous injection or intramusclar injection in a state of a solution, a suspension or an emulsion, etc. It can be provided in a dried state which can be liquefied by adding a suitable vehicle before using. Such liquid agents may contain suspending agents such as methyl cellulose, emulsifiers such as lecithin, antiseptics such as methyl-p-hydroxybenzoate and stabilizers or buffers which do not have an adverse influence upon an immune function of humans and animals, etc. It is possible to use physiological saline as an aqueous medium and vegetable oils such as sesame oil, etc., mineral oils such as paraffin, etc., vegetable and animal oils such as squalene, etc., and propylene glycol, etc. as a non-aqueous medium. Further, such a liquid agent may contain suitable adjuvants for promoting immunity. For example, there are Freund's complete adjuvant, saponin for animals and aluminium hydroxide for humans, etc.

The above described anticancer agent can be administered for patients with cancers at a time or several times over a long period of time in order to remedy the disease, and it can be administered for persons who are in danger of taking a cancer in order to prevent the development of the cancer.

Since both the A-GRA and the thermally denatured GRA have a low toxicity such that $LD_{50}$ (when administered intraperitoneally to a mouse) is 500 mg/kg or more as saccharide, they can be administered in an amount of a wide range. Accordingly, the amount of A-GRA or thermally denatured GRA in the anticancer agent is not particularly restricted, but it is generally preferred to be in a range of 0.001 to 100 µg/ml as saccharide. The amount of administration depends upon the state of disease, age and sexuality, but it is preferred to administer at a time to several times in an amount of 0.001 to 1000 µg/kg/day.

Further, killer cells obtained as described above are useful as an anticancer agent, too. Such an anticancer agent is preferred to be used as an injection preparation together with a vehicle used for this kind of blood agents. Though the vehicle is not particularly restricted, it is preferred to use those which have the same toxicity as blood, preferably physiological saline. In carrying out production of the agent, it is preferred that, after the killer cells are washed sufficiently with physiological saline to remove the above described medium, they are suspended in a vehicle.

The concentration of killer cells in the above described agent is not particularly restricted, but it is generally preferred in a range of $10^5$ to $10^9$ cells/ml. Further; toxicity of killer cells is not observed when they are administered in an amount of $10^8$ cells/mouse (intraperitoneally). The dosage depends upon the state of disease, age and sexuality, but it is preferred to administer at a time to several times in an amount of $10^5$ to $10^{12}$ cells/kg/day.

In the following, the present invention is illustrated with reference to examples, reference examples, test examples and comparative examples, but the present invention is not limited thereto.

REFERENCE EXAMPLE 1

(Localization of GRA)

(1) Production of FITC-labeled lectin (PNA-FITC):

10 mg of peanut lectin (PNA, produced by EY Co.) was dissolved in 2 ml of a 0.01M-phosphate buffer solution (pH=7.2) containing 0.85% of NaCl. 2 mg of FITC (produced by Sigma Co.) was dissolved in 1 ml of a 0.5M-bicarbonate buffer solution (pH=9.0), and 0.5 ml of the resulting solution was added to the above described buffer solution containing PNA. After stirring at room temperature for 2 hours, separation was carried out with Sephadex G25 (10 mm x 300 mm, produced by Pharmacia Co.) to collect a first peak. Ratio F/P = 1.0.

(2) Production of FITC-labeled lectin (DBA-FITC):

DBA-FITC was obtained using DBA (produced by EY Co.) by the same manner as in (1) above. The ratio F/P = 0.9.

(3) GRA localization in various kinds of cancer cells:

After 1 x 10$^6$ cells of various kinds of cancer cells were washed three times with a 0.05M-tris hydrochloric acid buffer solution (pH=7.2) containing 0.85% of NaCl by a centrifugal method, PNA-FITC obtained in (1) above or DBA-FITC

- 20 -

obtained in (2) above or SBA-FITC (produced by EY Co.) (200 µg/ml) was added in an amount of 100 µl thereto, and the resulting mixture was allowed to stand at room temperature for 30 minutes to carry out the reaction. After conclusion of the reaction, the cells were washed three times with a 0.01M phosphate buffer solution (pH=7.2) containing 0.85% of NaCl. The cells were put on a glass slide and examined by a fluorescence microscope.

Results obtained are shown in Table 1. The specimens of cancer cells were all known and could be available in the First Pathology Section, Medical Department, Niigata University.

## Table 1

| Specimen of Cancer Cells | | GRA Positivity (%) | |
|---|---|---|---|
| | | PNA-FITC | DBA-FITC (SBA-FITC) |
| Raji | (Burkitt lymphoma) | 98.3 | 1.4 |
| Daudi | (Burkitt lymphoma) | 93.1 | 5.2 |
| BT-1 | (Burkitt lymphoma) | 50.1 | 0 |
| P-12 | (T-cell lymphoma) | 44.3 | 6.7 |
| MOLT | (T-cell leukemia) | 0.6 | 4.8 |
| Fujimaki | (B-cell lymphoma) | 19.1 | 5.3 |
| Oda | (IgD myeloma) | 0.6 | 10.0 |
| QG-56 | (lung cancer. sq.) | 70.4 | 2.0 |

| PC-1 | (lung cancer. sq.) | 78.4 | 0.4 |
|---|---|---|---|
| PC-3 | (lung cancer. adeno.) | 77.1 | 0 |
| QG-90 | (lung cancer. small cell) | 68.0 | 0 |
| PC-13 | (lung cancer. large cell) | 17.0 | 0 |
| MK-2 | (stomach cancer. por.) | 63.7 | 0.1 |
| KATO-III | (stomach cancer. sig.) | 57.3 | 0 |
| MKN-45 | (stomach cancer. por.) | 1.0 | 40.3 |
| MKN-1 | (stomach cancer. adsq.) | 4.6 | 0.4 |
| MKN-28 | (stomach cancer. tubl) | 0.4 | 0.1 |
| MKN-74 | (stomach cancer. tubl) | 0.5 | 0 |
| MGH-U1 | (urinary bladder ca.) | 37.4 | 0 |
| KU-2 | (urinary bladder ca.) | 4.5 | 21.4 |
| T-24 | (urinary bladder ca.) | 14.6 | 0 |
| NBT-2 | (urinary bladder ca.) | 13.1 | 1.0 |
| NRC-12 | (renal cancer) | 23.9 | 0 |
| KU-1 | (renal cancer) | 3.3 | 0.6 |
| Kuramochi | (ovarian cancer) | 80.0 | 0 |
| NB-1 | (neuroblastoma) | 50.9 | 1.7 |
| YT-nu | (neuroblastoma) | 3.6 | 0.5 |
| TGW-nu-I | (neuroblastoma) | 4.1 | 0 |
| TGW-nu-II | (neuroblastoma) | 2.0 | 1.0 |
| GOTO | (neuroblastoma) | 0.5 | 0 |
| ITO | (embryonal carcinoma) | 96.9 | 12.3 |
| NEC-8 | (embryonal carcinoma) | 44.6 | 0 |
| SCH | (chriocarcinoma stomach) | 14.6 | 3.1 |

| GCH | (chriocarcinoma uterus) | 5.4 | 0 |
| YN-1 | (rhabdomyosarcoma) | 5.7 | 1.7 |
| X5563 | (mouse myeloma) | 92.0 | 0 (90.6) |
| MH134 | (mouse ascites liver cancer) | 18.6 | 0 (6.4) |

(4) Localization of GRA in various kinds of cancer cells (operated sample):

A cancer tissue obtained from an operated sample of a patient with cancer was allowed to pass through a stainless steel mesh (#150) to obtain a cell suspension. It was washed twice with a 0.01M tris-hydrochloric acid buffer solution (pH=7.4) containing 2 mM of $CaCl_2$, 2 mM of MgCl and 0.85% of NaCl. $5 \times 10^5$ cells were suspended in 100 µl of the above-described buffer solution, and 100 µl of PNA-FITC or DBA-FITC (200 µg/ml) was added thereto followed by incubating at room temperature for 20 minutes. After conclusion of the reaction, cells were washed three times with 0.01M phosphate buffer solution (pH=7.2) containing 0.85% of NaCl (referred to as "PBS", hereinafter). Thereafter, they were put on a glass slide and examined by a fluorescence microscope. Results are shown in Table 2. The operated samples of patients with cancers were available in Kansei Medical College. In the Table 2, localization of GRA is shown as follows.

+:  GRA is expressed on the surface of cells.

-:  GRA is not expressed on the surface of cells.


## Table 2

| Specimen of Cancer Tissue | Localization of GRA | |
| --- | --- | --- |
| | PNA-FITC | DBA-FITC |
| Stomach cancer | + | + |
| " | + | + |
| " | - | - |
| " | + | - |
| " | + | + |
| " | + | - |
| Mastocarcinoma | + | - |
| " | + | - |
| " | + | - |
| " | + | + |
| Colon cancer | + | + |
| " | - | + |
| Esophageal cancer | + | - |
| Liver cancer | - | + |


### REFERENCE EXAMPLE 2

Production of Lectin Receptor:

(1)  Production of Insoluble Lectin (PNA-Sepharose):

After 3 g of CNBr-activated Sepharose 4B (produced by Pharmacia Co.) was sufficiently washed with 1 mM-HCl, it was suspended in 200 ml of 0.1M sodium hydrogen carbonate (pH=8.5). 5 ml of a 0.01M phosphate buffer solution (pH= 7.7) containing 20 mg of PNA was added thereto and the reaction was carried out at 25°C for 2 hours with stirring at times to obtain PNA-Sepharose.

(2)      DBA-Sepharose was obtained by the same procedures as in (1) above, except that DBA was used instead of PNA.

(3)      $1.3 \times 10^8$ cells of BT-1 (Burkitt lymphoma) were washed three times with physiological saline, and 30 ml of a 0.01M tris-hydrochloric acid buffer solution (pH=7.4) containing 2% of "Triton X-100" (produced by Wako Pure Chemical Industries, Ltd.), 0.85% of NaCl, 2 mM of $CaCl_2$ and 2 mM of $MgCl_2$ was added thereto. The resulting mixture was stirred at 4°C for 15 minutes. It was then subjected to ultracentrifugation at 100,000 X g for 2 hours. Of 28 ml of the ultracentrifugal supernatant, 14 ml aliquot was subjected to affinity chromatography (diameter: 0.5 cm; length: 1 cm) with PNA-Agarose beads (produced by Maruzen Co.) equilibrated with a tris-hydrochloric acid buffer solution (pH=7.4) containing 0.1% of Triton X-100, 0.85% of NaCl, 2 mM of $CaCl_2$ and 2 mM of $MgCl_2$. After washing with the above described buffer solution, elution was carried out with a 0.01M tris-hydrochloric acid buffer solution (pH=7.4) containing 0.1M lactose, 0.5% of NaCl, 2 mM of $CaCl_2$, 2 mM of $MgCl_2$ and 0.1% of Triton X-100, and the eluted part was dialyzed against a 0.01M tris-hydrochloric acid buffer solution containing 0.85% of NaCl, 2 mM of $MgCl_2$ and 2 mM of $CaCl_2$ for 48 hours to obtain 17 ml of a solution of lectin receptor. As a result of measuring the amount of protein thereof by a Folin-Lowry method and the amount of saccharide thereof by a phenol sulfate method, the amount of protein was 644 g and the amount of saccharide was 120 g. Hereinafter, it is referred to as "Receptor 1".

(4) $1 \times 10^{10}$ cells of $C_3H$.mouse mastocarcinoma (MMT) were washed three times with physiological saline. Then, 30 ml of a 0.01M tris-hydrochloric acid buffer solution (pH= 7.4) containing 2% of Triton X-100, 0.85% of NaCl, 2 mM of $CaCl_2$ and 2 mM of $MgCl_2$ was added thereto and the mixture was stirred at 4°C for 30 minutes. Thereafter, it was subjected to ultracentrifugation at 100,000 X g for 2 hours, and the resulting supernatant was dialyzed for a night against a 0.01M tris-hydrochloric acid buffer solution (pH= 7.4) containing 0.85% of NaCl, 2 mM of $CaCl_2$ and 2 mM of $MgCl_2$. The resulting dialysate was concentrated to 3 ml by Immersible-CXultra-filters (produced by Millipore Co.) and 1 ml of it was subjected to affinity chromatography with PNA-Sepharose equilibrated with a tris-hydrochloric acid buffer solution (pH=7.4) containing 0.005% of Triton X-100, 0.85% of NaCl, 2 mM of $CaCl_2$ and 2 mM of $MgCl_2$ (diameter: 0.5 cm; length: 2 cm). After sufficiently washing with the same buffer solution, elution was carried out with a 0.01M tris-hydrochloric acid buffer solution. (pH=7.4) containing 0.1M of lactose, 0.85% of NaCl, 2 mM of $CaCl_2$, 2 mM of $MgCl_2$ and 0.005% of Triton X-100, and the eluted part was dialyzed for 48 hours against a 0.01M tris-hydrochloric acid buffer solution (pH=7.4) containing 0.85% of NaCl, 2 mM of $CaCl_2$ and 2 mM of $MgCl_2$ to obtain 2 ml of a solution of lectin receptor. The amount of protein thereof was 156 µg and the amount of saccharide thereof was 94 µg. Hereinafter, it is referred to as "Receptor M-1".

(5)     About 120 g (wet weight) of KATO-III cells was homogenized in 100 ml of PBS by means of a mill (Waring blender: produced by Nippon Seiki Co., Ltd.). The precipitate obtained by centrifugal separation (100,000 g x 1 hour) was added to 100 ml of a 0.01M tris-hydrochloric acid buffer solution (pH=7.6) containing 2% of Triton X-100 and 0.15M of NaCl with stirring. The supernatant obtained by centrifugal separation (100,000 g x 1 hour) was subjected to affinity chromatography (diameter: 0.8 cm; length: 15 cm) with PNA-Sepharose equilibrated with 0.01M tris-hydrochloric acid buffer solution (pH=7.6) containing 0.015% of Triton X-100 and 0.15M of NaCl. After washing with 50 ml of the same buffer solution, elution was carried out with the same buffer solution containing 0.1M of lactose, and the elute was dialyzed against a 0.85% aqueous solution of NaCl to obtain a solution of lectin receptor. It was concentrated with Sephadex (produced by Pharmacia Co.) and preserved at -20°C. The amount of protein: 2.0 mg. The amount of saccharide: 0.8 mg. Hereinafter, it is referred to as "Receptor 2".

(6) Lectin receptors shown in Table 3 were obtained by the same manner as in (5) above, respectively.

Table 3

| Receptor | Raw Material | Amount of Protein (mg) | Amount of Saccharide (mg) |
|---|---|---|---|
| 3 | BT-1, 33 g | 0.5 | 0.09 |
| 4 | Mastocarcinoma (operated sample), 5 g | 0.24 | 0.5 |
| 5 | QG-56, 24 g | 0.6 | 0.38 |
| 6 | QG-90, 26 g | 1.0 | 0.54 |
| 7 | Raji, 29 g | 0.78 | 0.45 |
| M-2 | MMT, 200 g | 11.3 | 28.4 |
| M-3 | Lewis lung cancer (LLC), 14.4 g | 0.06 | 0.07 |
| M-4 | Ascites liver cancer (MH134), 85 g | 0.65 | 0.35 |
| M-5 | Myeloma (X5563), 25 g | 0.56 | 0.23 |

(7) A lectin receptor was obtained by the same procedure as in (5) above, except that about 29 g of MKN-45 was used instead of KATO-III, a DBA-Sepharose column was used instead of the PNA-Sepharose column, and elution was carried out with N-acetylgalactosamine instead of lactose. Amount of protein: 0.03 mg. Amount of saccharide: 0.01 mg. Hereinafter, it is referred to as "Receptor 8".

(8) 5 ml of Receptor 3 obtained in (6) above was subjected to treating with a DBA-Sepharose column, and elution was carried out with a 0.01M tris-hydrochloric acid buffer solution containing 0.015% of Triton X-100, 2 mM of $MgCl_2$, 2 mM of $CaCl_2$ and 0.85% of NaCl to obtain fractions of every 4 ml. Then, elution was carried out with the above described buffer solution containing 0.1M N-acetylgalactosamine to obtain a solution of lectin receptor. Hereinafter, it is referred to as "Receptor 3-C". Further, the above described fractions No. 1 to 3 are referred to as Receptor 3-A", and the fractions No. 4 to 12 are referred to as "Receptor 3-B".

(9) SDS gel electrophoresis of each lectin receptor obtained as described above was carried out according to the process by Fairbanks et al (Briochemistry, Vol. 10, p. 2606 (1971)). Results are shown in Figures 1 to 5.

In the drawings, each number is as follows.

In Figure 1 and Figure 2:

1 ..... Standard

2 ..... Receptor M-3

3 ..... Receptor 7

4 ..... Receptor 1

5 ..... Receptor 2

In Figure 3:

1 ..... Standard

2 ..... Receptor M-2

3 ..... Receptor 6

4 ..... Receptor 5

In Figure 4:

1 ..... Receptor M-4

2 ..... Receptor M-5

3 ..... Standard

In Figure 5:

1 ..... Receptor 3

2 ..... Receptor 3-A

3 ..... Receptor 3-B

4 ..... Receptor 3-C

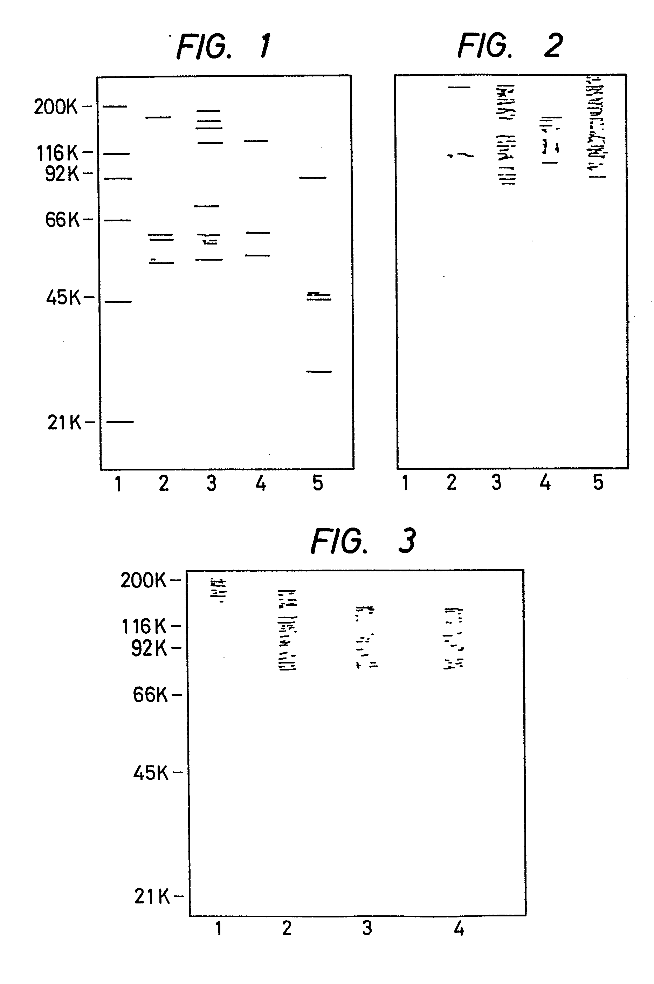

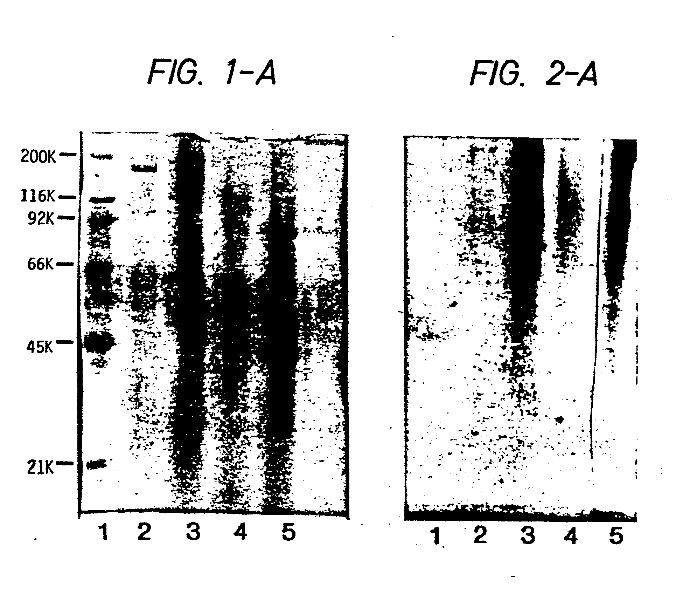

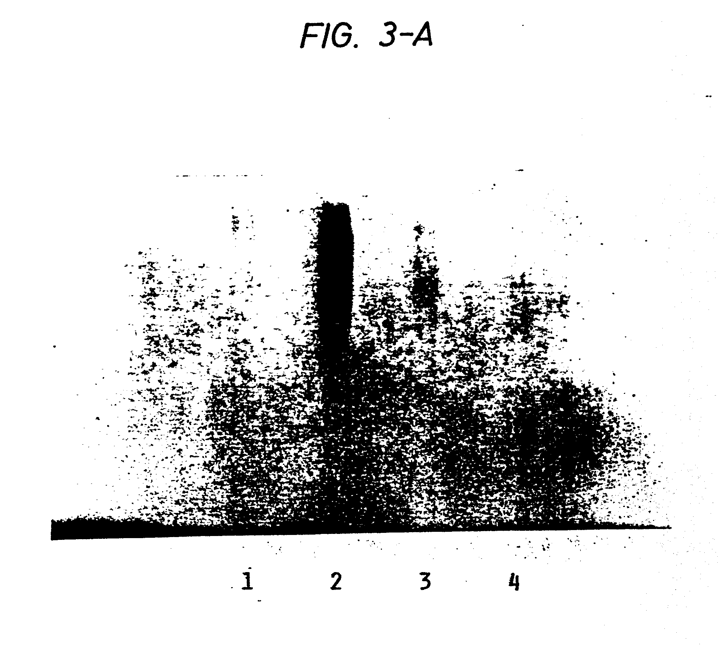

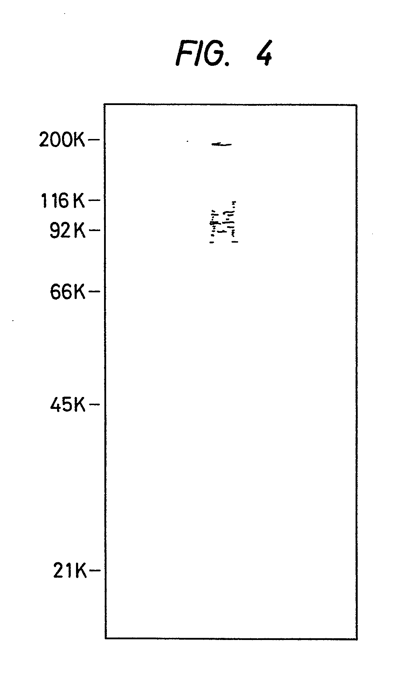

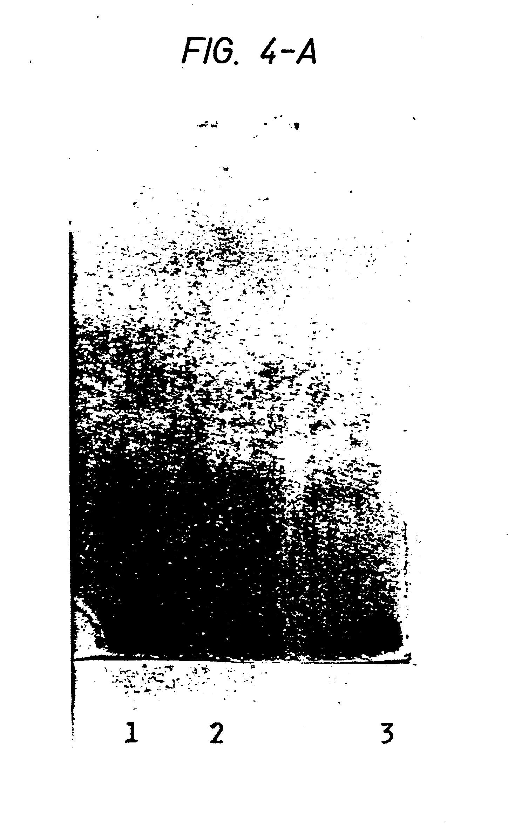

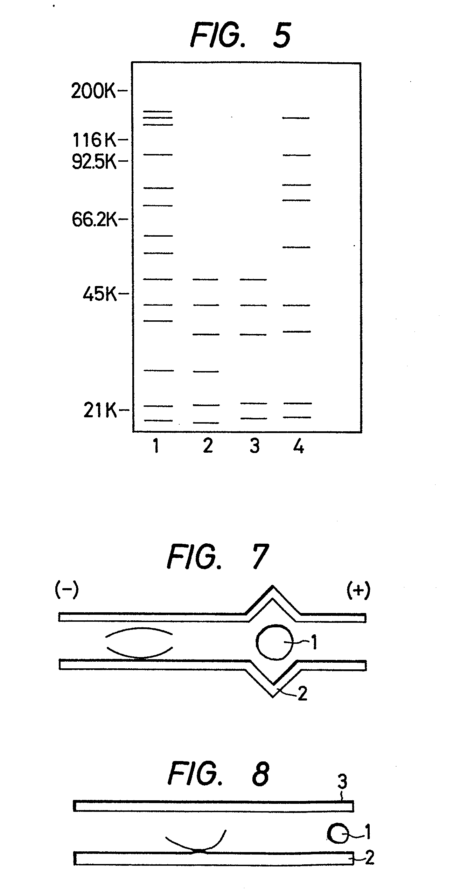

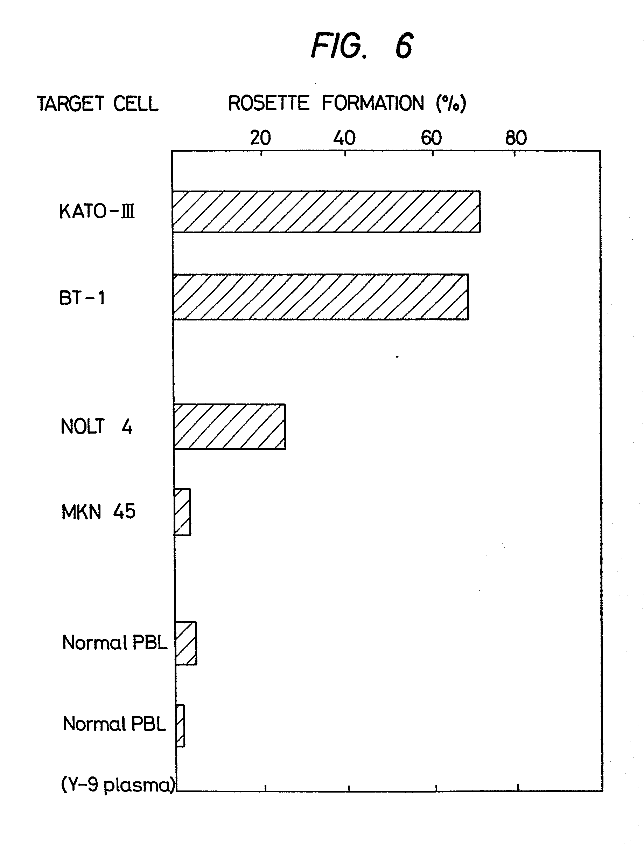

Further, Figures 1 and 5 are drawings representing electrophoretic diagram detected by a dyeing reaction of protein by means of a C.B.B. method (Biochemistry, Vol. 10, p. 2606 (1971)) and Figures 2 to 4 are representing electrophoretic diagram drawings detected by a dyeing reaction of saccharide by means of a Pas method (Anal. Biochem., Vol. 30, 148 (1962)).

In each drawing, as the standard, the following standard materials produced by Biorad Lab. Co. (U.S.A.) were used.

200 (K daltons) : Myosin

116 ( " ) : -Glucosidase

925 ( " ) : Phosphorylase

662 ( " ) : BSA

45 ( " ) : Ovalbumin

215 ( " ) : Soybean trypsin inhibitor

Further, photographs corresponding to Figures 1 to 4 are shown as Figures 1A to 4A.

## REFERENCE EXAMPLE 3

(ORBC-Protein A)

ORBC was centrifugally washed three times (2000 rpm, 10 minutes) with a 0.01M phosphate solution (pH=7.2) containing 0.85% of NaCl to obtain $5 \times 10^9$ cells by precipitation. 1 mg/ml of physiological saline containing 0.45 ml of Protein A (Pharmacia Co.) and 1 mg/ml of physiological saline containing 0.5 ml of $CrCl_3 \cdot 6H_2O$ were added thereto, and the mixture was stirred at room temperature for 10 minutes. After the cells were washed with two or three times with RPMI-1640 medium, they were suspended in 25 ml of the same medium ($2 \times 10^8$ cells/ml) and preserved at 4°C.

## REFERENCE EXAMPLE 4

(Measurement of GRA Antibody)

After incubation cancer cell, Daudi, was centrifugally washed twice (1,000 rpm, 10 minutes) with the medium RPMI-1640, the sample was prepared so as to have 3 x $10^6$ cells/ml in the same medium. 100 µl of it was put in a test tube, and 100 µl of a serum of a patient with cancer was added thereto and the reaction was carried out at 4°C for 1 hour. After the cells were washed twice with the same medium, 100 µl of ORBC-Protein A obtained in Reference Example 3 was added to the precipitate. After stirred, it was subjected to centrifugation at 1,000 rpm for 5 minutes. Thereafter, it was slightly stirred and fixed and dyed with glutaraldehyde and Coomassie Brilliant Blue (CBB). Then rosette formation of the ORBC-Protein A to the incubated cells was measured. Results are shown in Table 4.

Table 4

| Sample No. | Cancer | Rosette Formation (%) |
|---|---|---|
| 1 | Esophageal cancer | 76 |
| 2 | Mastocarcinoma | 28 |
| 3 | " | 10 |
| 4 | " | 1 |
| 5 | " | 10 |
| 6 | " | 90 |
| 7 | " | 85 |
| 8 | Rectal cancer | 50 |
| 9 | " | 15 |

| 10 | " | 97 |
|----|---|----|
| 11 | Stomach | 65 |
| 12 | " | 45 |
| 13 | " | 45 |
| 14 | " | 20 |
| 15 | " | 5 |
| 16 | " | 35 |
| 17 | Colon cancer | 20 |
| Normal cells (10 men) | | 0 |

## REFERENCE EXAMPLE 5

Rosette formation was measured by the same manner as in Reference Example 4, except that various kinds of cancerous cell having each a different property of being bound to lectin were used instead of Daudi. As the serum of patient, that of No. 11 in Table 4 was used. The results are shown in Figure 6. It is understood therefrom that the antibody strongly reacts with PNA receptors.

## REFERENCE EXAMPLE 6

Receptor 1 obtained in Reference Example 2 in an amount of 5 g as protein together with a Freund's complete adjuvant was subcutaneously administered to a rabbit (2 to 3 kg) once every two weeks to immunize the animal. After immunizing three times, the blood was collected, and an antiserum was collected by centrifugal separation to obtain a GRA antibody (antibody-I).

## REFERENCE EXAMPLE 7

(1)    A blood was collected from the patient No. 10 having a high rosette formation in Reference Example 4 and an antiserum was collected by centrifugal separation to obtain a GRA antibody (antibody-II).

(2)    To 9 ml of Antibody-II obtained in (1) above, 6 ml of a saturated solution of ammonium sulfate was dropwise added. After stirred at room temperature for 1 hour, the mixture was subjected to centrifugal separation (3,000 rpm, for 30 minutes) to obtain a precipitate. After the precipitate was dissolved in 6 ml of PBS, it was dialyzed with 5 ℓ of PBS at 4°C for 24 hours to obtain a GRA antibody (Antibody-III) containing 12 mg protein/ml.

## REFERENCE EXAMPLE 8

(Reactivity of GRA antibody)

(a)    Using Receptor 2 obtained in Reference Example 2 as an antigen, reactivity thereof with the GRA antibody (Antibody-II) obtained in Reference Example 7 was examined by an immuno-electrophoretic method. Namely, 15 µl of the antigen (160 µg protein/ml) was put in a hole of a 1% Agarose plate to carry out electrophoresis at 70V for 60 minutes. 200 µl of Antibody-II was put in a groove to allow to react at room temperature and room humidity for 24 hours. The result was as shown in Figure 7, where a sedimentation line was observed. In Figure 7, numeral 1 is a hole and 2 is a groove.

(b)      Reactivity of the same antigen with the  antibody as in (a) was examined by a precipitation method using a capillary (refer to "Hyojun Rinsho Kensaho" Igakushoin, 2nd edition, pages 342-345 (1967)). Namely, Receptor  2 was put in a capillary and Antibody-II was superposed thereon. They were allowed to stand at room temperature for 24  hours. As the result, a precipitate was observed on the  boundary face between the antigen and the antibody. When using a serum of a normal person instead of the antibody,  a  precipitate was not observed.

(c)  Block Test (PNA)

100 µl of a PBS solution of  PNA  having  a suitable concentration was put in a test tube  containing  BT-1  (3 x $10^5$ cells/ml RPMI-1640) to incubate at 4°C for  20  minutes. After centrifugally washed twice with  the  medium RPMI-1640 (1,000 rpm, for 10 minutes), the cells were  suspended again in 100 µl of the above described medium. After adding 100 µl of a PBS 10  time-diluted solution of Antibody-II obtained in Reference Example 7 to  the  suspension  and incubating it at 4°C for 1 hour,  it  was  washed  three times with the above described medium.  The  cells were suspended again in 100 µl of the above described medium, and 100 µl of ORBC-protein A was added thereto.  Then,  rosette formation was measured by the same manner as in Reference  Example  4.

The results were as shown in Table 5, wherein a blocking effect for binding by PNA was observed.

### Table 5

| Concentration of PNA (µg/ml) | 0 | 250 | 500 | 1000 |
|---|---|---|---|---|
| Rosette Formation (%) | 78.5 | 62.3 | 42.4 | 23.3 |

(d)  Block Test (Lectin Receptor)

To 0.1 mol of a PBS of Receptor 1 obtained in Reference Example 2 having a suitable concentration, 0.1 ml of a PBS diluted solution containing 10 µg/ml of Antibody-III obtained in Reference Example 7 was added. After the reaction at 37°C for 1 hour, Daudi 3 x $10^5$ cells/0.1 mol RPMI-1640 was added to carry out incubation at 4°C for 1 hour. The cells were centrifugally washed three times (1,000 rpm, 10 minutes) with RPMI-1640. The resulting cells were suspended again in 100 µl of the above described medium, and rosette formation was measured by the same manner as in Reference Example 4. The results were as shown in Table 6, wherein a blocking effect for binding by lectin receptor was observed.

## Table 6

| Concentration of Lectin Receptor (μg/ml) | 0 | 2.2 | 4.4 | 8.8 |
|---|---|---|---|---|
| Rosette Formation (%) | 43.5 | 4.0 | 1.9 | 0.2 |

(e) Using Receptor 1 obtained in Reference Example 2 as an antigen, reactivity of Antibody-I was examined by the same manner as in (a) above. The results are shown in Figure 8. In the drawing, 1 is a hole, 2 is a groove for Antibody-I, and 3 is a groove for a serum of a normal rabbit. As shown in the drawing, a sedimentation line was observed in Antibody-I only.

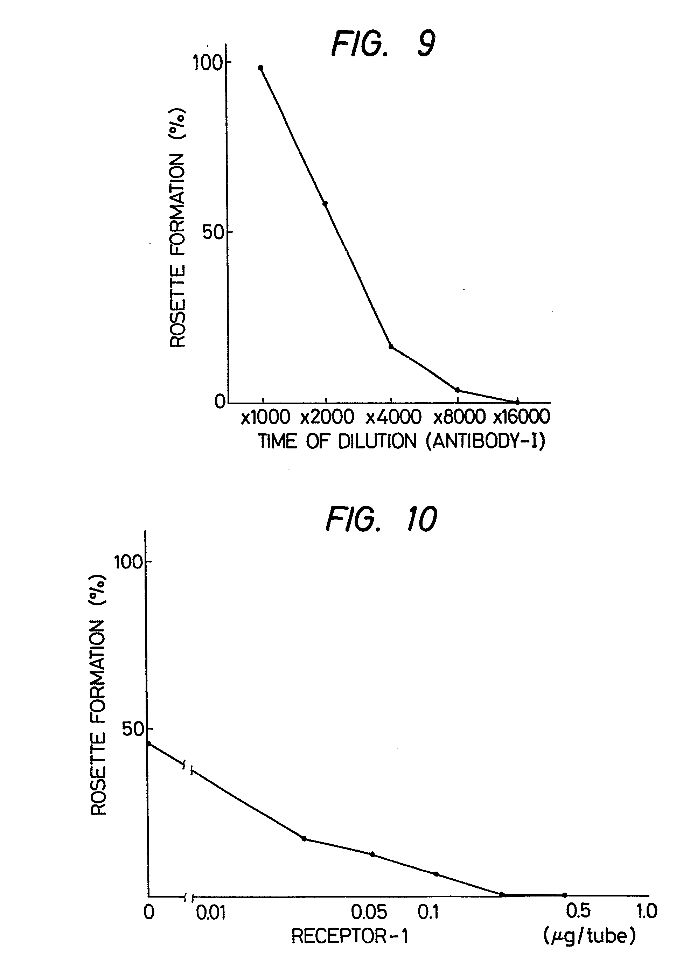

(f) Rosette formation was measured by the same manner as in Reference Example 4, except that 100 μl of Antibody-I diluted four times with PBS was used instead of the patient serum. The results obtained are shown in Fig. 9.

(g) To 100 μl of a PBS solution of Receptor 1 obtained in Reference Example 2 having a suitable concentration, 100 μl of a PBS 700 time diluted solution of Antibody-I was added. After reaction at 37°C for 90 minutes, Daudi $3 \times 10^5$ cells/0.1 ml RPMI-1640 medium was added and incubation was carried out at room temperature for 60 minutes. The cells were centrifugally washed three times (1,000 rpm, 10 minutes) with the medium RPMI-1640. The cells were suspended again in 100 μl of the above described medium, and rosette formation was measured by the same manner as in Reference Example 4. The results were as shown in Figure 10, wherein a blocking effect for binding by the lectin receptor was observed.

REFERENCE EXAMPLE 9

(a)      To 2 ml of Antibody-III obtained in Reference Example 7, a 0.1M aqueous solution of sodium hydrogen carbonate containing 5 g/15 ml of Bromocyan Sepharose (Pharmacia Co.) and 0.5M of NaCl (pH=8.3) was added, and the resulting mixture was stirred for 2 hours to obtain an insoluble carrier of GRA antibody.

(b)      An insoluble carrier of GRA antibody was obtained by the same manner as in (a) above using Antibody-I obtained in Reference Example 6.

EXAMPLE 1

(1)      About 40 g (wet weight) of KATO-III cells was homogenized in 50 ml of PBS by means of a mill (Waring blender). A precipitate obtained by centrifugal separation (100,000 g X, 1 hour) was added to 50 ml of a 0.01M tris-hydrochloric acid buffer solution (pH=7.6) containing 2% of Triton X-100 and 0.5M of NaCl with stirring. The supernatant obtained by centrifugal separation (100,000 x g, 1 hour) was introduced into a column (diameter: 0.8 cm; length: 15 cm) of GRA antibody-Sepharose (the carrier obtained in Reference Example 9-(a)) equilibrated with a 0.01M tris-hydrochloric acid buffer solution (pH=7.6) containing 0.015% of Triton X-100 and 0.15M of NaCl. After washing with the same buffer solution, elution was carried out with the same buffer solution containing 0.1M of lactose, and the elute was dialyzed with a 0.85% aqueous solution of NaCl to obtain a solution of A-GRA. Amount of protein: 0.45 mg. Amount of saccharide: 0.12 mg. Hereinafter, it is referred to as "GRA-1".

(2)    A solution of A-GRA was obtained by the same procedure as in (1) above, except that 20 g of BT-1 cells was used instead of the KATO-III cells and a carrier obtained in Reference Example 9-(b) was used as the GRA antibody-sepharose column. Amount of protein: 230 µg. Amount of saccharide: 40 µg. Hereinafter, it is referred to as "GRA-2".

(3)    After a column of 5 ml of a carrier obtained in Reference Example 9-(a) was washed with a 0.01M tris-hydrochloric acid buffer solution (washing solution having pH 7.6) containing 0.015% of Triton X-100, Receptor 2 obtained in the reference example was allowed to flow through it in an amount of 350 µg as protein to carry out affinity chromatography. After washing with a washing solution, 30 ml of lactose was allowed to flow through the column. The eluted fraction is hereinafter referred to as GRA-3 (amount of protein: 164 g). After further washing, elution was carried out with a 0.2M hydrochloric acid-glycine buffer solution (pH=2.7). The eluted fraction is hereinafter referred to as GRA-4 (amount of protein: 97 µg).

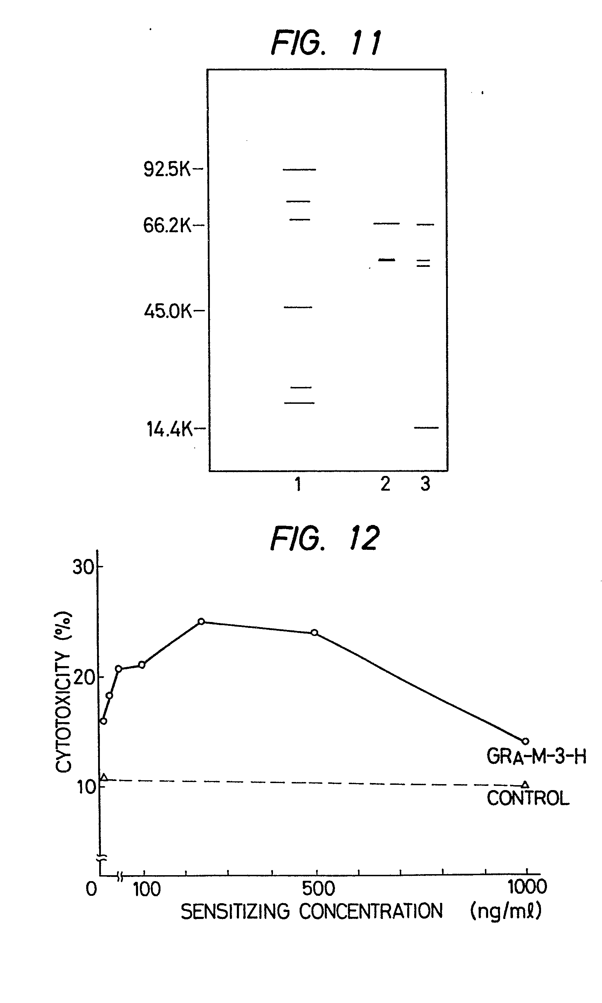

SDS electrophoresis patterns of each fraction obtained as described above obtained by the same manner as in Reference Examples 2 to 9 are shown in Figure 11.

The Figure 11 shows dyeing patterns by a C.B.B. method, wherein each number is as follows.

1 ..... Receptor 2

2 ..... GRA-3

3 ..... Gra-4

## EXAMPLE 2

A physiological saline containing 100 µg as protein of Receptor M-3 obtained in Reference Example 2 was heated to 100°C for 10 minutes in a boiling water bath to obtain a thermally denatured antigen. Hereinafter, it is referred to as "GRA-M-3-H". It was adjusted to have a concentration of 10 µg protein/ml and preserved at 4°C.

## EXAMPLES 3 TO 13

Thermally denatured GRA shown in the following Table 7 were obtained by the same manner as in Example 2, except that lectin receptors obtained in the Reference Example 2 and A-GRA obtained in Example 1 were used instead of Receptor-M-3.

- 40 -

## Table 7

| Example No. | Raw Material | Heating Condition | Thermally Denatured Antigen |
|---|---|---|---|
| 3 | Receptor 1 | 100°C, 20 minutes | GRA-1-H-1 |
| 4 | " 1 | 100°C, 10 minutes | GRA-1-H-2 |
| 5 | " 2 | 90°C, 20 minutes | GRA-2-H |
| 6 | " 4 | 120°C, 5 minutes | GRA-4-H |
| 7 | " 6 | 100°C, 10 minutes | GRA-6-H |
| 8 | " 8 | 100°C, 10 minutes | GRA-8-H |
| 9 | " 3-C | 110°C, 10 minutes | GRA-3-C-H |
| 10 | GRA-1 | 100°C, 10 minutes | GRA-1-HA |
| 11 | " -2 | 100°C, 10 minutes | GRA-2-HA |
| 12 | " -3 | 100°C, 10 minutes | GRA-3-HA |
| 13 | " -4 | 120°C, 5 minutes | GRA-4-HA |

## REFERENCE EXAMPLE 10

(Preparation of Lymphocyte)

(1) Human Peripheral Blood Lymphocyte (Human PBL)

50 ml each of blood samples obtained by heparin collection from a healthy person or patients with various cancers was subjected to centrifugal separation with "Ficollpack" (produced by Pharmacia Japan Co.) to obtain 5 x $10^7$ cells of peripheral blood lymphocyte.

(2) Mouse Spleen Lymphocyte

The spleen of C57BL/6 mouse (male, 6 weeks) was picked out and washed twice with the medium RPMI-1640. After it was loosened by an injector needle, it was filtered by a stainless steel mesh (No. 100) to remove fragments having a large size. After the cells filtered off was washed twice with the above described medium, they were centrifuged at 1,200 X g for 10 minutes to obtain $4 \times 10^7$ spleen lymphocytes.

### REFERENCE EXAMPLE 11

(1)     Mouse spleen lymphocytes obtained in Reference Example 10-(2) were adjusted with the medium RPMI-1640 containing 15% of FCS so as to be $2 \times 10^6$ cells/ml, and GRA-M-3-H obtained in Example 2 was added thereto so as to have a final protein content of 5, 25, 50, 100, 250, 500 or 1,000 ng/ml. After incubation at 37°C in a carbon dioxide gas incubator for 24 hours, they were washed twice with the above described medium to obtain killer cells. They were adjusted with the above described medium so as to be $2 \times 10^6$ cells/ml.

(2)     The cytotoxicity of the killer cells obtained in (1) above to cancerous cells was measured according to Single Cell Cytotoxic Assay (The Journal of Immunology, Vol. 128, No. 6, p. 2514-2521 (1982)). Namely, LLC cells were used as target cells. After washed, $5 \times 10^4$ cells of them and $2.5 \times 10^5$ cells of the above described killer cells (E/T=5) were mixed with 0.2 ml of the medium RPMI-1640 containing 10% of FCS. After incubated at room temperature for 5 minutes, the mixture was centrifuged at 1,000 rpm for 5 minutes, and 1% Agarose was added thereto so as to have a final concentration of 0.5%. On a Petri dish on which 1% Agarose was previously put, the above described cells were placed and slightly blended, and incubation was carried out at 37°C for 1 hour. After addition of 0.5 ml of 0.2% Trypan Blue, they were allowed to stand for 10 minutes. Thereafter, they were washed twice with physiological saline. The number of dead target cells to which the killer cell was attached (A) was measured by an inverted phase contrast microscope, and the cytotoxicity of killer cells to cancerous cells was calculated from the following formula.

$$\text{Cytotoxicity (\%)} = \frac{A \times 100}{\text{Number of the total target cells}}$$

The results are shown in Figure 12. In the Fig. 12, the ordinate indicates the cytotoxicity and the abscissa indicates the amount of protein (concentration) of GRA-M-3-H used when inducing the killer cells. Further, the results obtained by a measurement using lymphocytes obtained by the same procedures as in (1) without using GRA-M-3-H are shown as a control. The cytotoxicity of the control was 10.7%.

As shown in Figure 12, the thermally denatured antigen of the present invention is effective in a wide range, because it has a low dependency on the concentration when forming cell mediated immunity (induction of killer cells), and it is understood that it has a great effect.

REFERENCE EXAMPLE 12

(1)     GRA-M-3-H obtained in Example 2 was adjusted with physiological saline so as to have a protein content of 100 ng/ml. Hereinafter, it is referred to as "Anticancer Agent No. 1".

(2)     $1 \times 10^4$ of LLC cells derived from C57BL/6 mouse were injected to C57BL/6 mouse (Charles liver, male, 5 weeks) from a vein of the tail. After 3 days, 6 days or 9 days from the injection, the above described anticancer Agent No. 1 was administered to the animal in an amount of 1 ml/mouse.day from a vein of the tail for 3 days. After 22 days from the injection of LLC cells, the lung was picked up and presence or absence of implanted LLC on the lung was visually observed and the weight of the lung was measured. As controls, a group to which the anticancer agent was not administered and normal mice were used. Results are shown in Table 8. It is confirmed from Table 8 that administration of the thermally denatured antigen of the present invention clearly causes tumor rejection or suppression of tumor growth.

Table 8

| Administration Day (day) | Mouse No. | Weight of Lung (g) | Presence or Absence of Implantation |
|---|---|---|---|
| No administration | 1 | 0.423 | + |
| | 2 | 0.305 | + |
| | 3 | 0.215 | + |
| | 4 | 0.239 | + |
| | 5 | 0.571 | + |
| 3, 4, 5 | 1 | 0.194 | − |
| | 2 | 0.182 | − |
| | 3 | 0.177 | − |
| | 4 | 0.359 | + |
| | 5 | 0.176 | − |
| 6, 7, 8 | 1 | 0.273 | − |
| | 2 | 0.319 | + |
| | 3 | 0.238 | − |
| | 4 | 0.199 | − |
| | 5 | 0.225 | + |
| 9, 10, 11 | 1 | 0.203 | − |
| | 2 | 0.174 | − |
| | 3 | 0.185 | − |
| | 4 | 0.200 | − |
| | 5 | 0.192 | − |
| Normal | 1 | 0.181 | − |
| | 2 | 0.183 | − |
| | 3 | 0.170 | − |
| | 4 | 0.177 | − |
| | 5 | 0.175 | − |

REFERENCE EXAMPLE 13

(1)     Peripheral blood lymphocytes obtained by the same manner as in Reference Example 10-(1) from a patient with (small cell cancer of lung, male, 67 years old) were adjusted with the medium RPMI-1640 containing 15% of FCS so as to have $2 \times 10^6$ cells/ml, and GRA-6-H obtained in Example 7 was added thereto so as to have a final protein content of 100 ng/ml. After they were incubated at 37°C in a carbon dioxide gas incubator for 24 hours, the lymphocytes were washed twice with the above described medium to obtain killer cells. They were adjusted with the above described medium so as to have a population of $2 \times 10^6$ cells/ml.

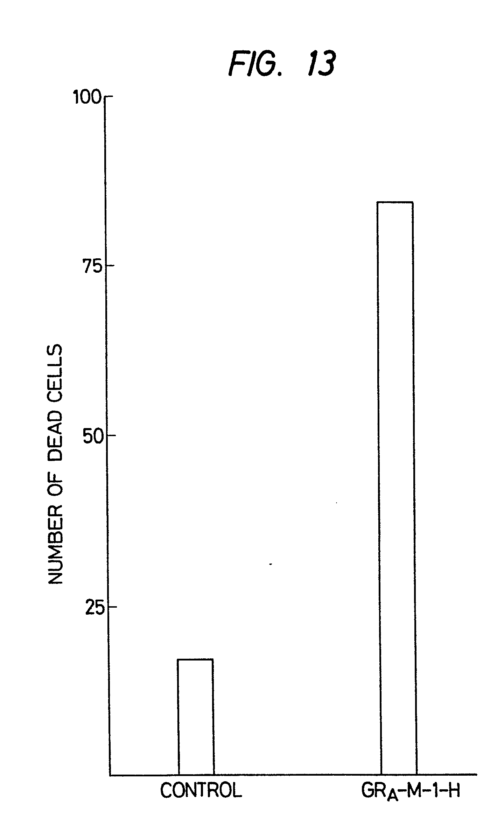

(2)     The cytotoxicity of the above described killer cells to cancerous cells was examined using QG90, which was an incubated strain of a small cell lung cancer of the same kind as the patient, as target cells. Namely, QG90 was incubated on a 96 hole flat bottom microplate to multiply till it became a monolayer, which was used as target cells. To the cells, 100 μl of the above described killer cells was dropwise added slowly, and incubation was carried out for 1 hour. After the supernatant was removed, 5 μl of a 5% Eosine solution was added to dry the cells followed by allowing them to stand for 3 minutes. After dyeing, the cells were washed 5 times with the medium RPMI-1640 containing 10% of FCS. Then, the number of the dead cancerous cells per well was measured by microscopic observation. The results obtained are shown in Figure 13. In Fig. 13, the ordinate indicates the number of dead cancerous cells. Further, lymphocytes obtained by the same procedures as in (1) above without using GRA-6-H was used as a control.

It is understood from Figure 13 that the thermally denatured antigen of the present invention has a very high induction activity for killer cells.

REFERENCE EXAMPLE 14

Ability of formation of cell mediated immunity (induction of killer cells) of the thermally denatured antigen of the present invention in the living body was examined using Macaca fuscata which is primates closest to Homo sapiens.

Namely, GRA-6-H was administered into a monkey of Macaca fuscata (Nippon Primates Co.) by intradermally administration at a dosage of 200 ng protein/monkey. After 3, 6, 24, 48 and 72 hours from administration, 5 ml of blood was collected from a femoral vein of the monkey using a heparin-treated syringe. Then lymphocytes were separated by the difference of specific gravity using a specific gravity separating solution SMF (produced by JIMRO), and they were adjusted with the medium RPMI-1640 so as to be $2 \times 10^6$ cells/ml. Then, the cancerous cell cytotoxicity of the lymphocytes was measured. This activity test was carried out using target cells (QG90) obtained by the same manner as in Reference Example 13-(2). Likewise, 200 μl of each lymphocyte solution was added dropwise. After incubation at 37°C in a carbon dioxide gas incubator for 90 minutes, dyeing was carried out using Eosine, and the number of dead cells was measured. The results are shown in the following Table 9. In Table 9, the number of dead cells is shown as an average ± SD per well. Further, the passage time: 0 hour means a control wherein lymphocytes which were collected just before administration of GRA-6-H were used.

Table 9

| Passing Time (Hour) | Number of Dead Cells | Well Number |
| --- | --- | --- |
| 0 | 0 | 3 |
| 3 | 0 | 3 |
| 6 | 37.6 + 10.4 | 6 |
| 24 | 60.5 + 13.7 | 6 |
| 48 | 13.0 + 8.8 | 4 |
| 72 | 15.5 + 4.6 | 6 |

It is understood from Table 9 that strong cell mediated immunity is formed in the living body by sensitization with the thermally denatured antigen of the present invention.

REFERENCE EXAMPLE 15

In Reference Example 11-(1), GRA-8-H, GRA-1-HA, GRA-2-HA, GRA-3-HA or GRA-4-HA, instead of GRA-M-3-H, was used so as to have a final amount of 250 ng protein/ml to produce killer cells, respectively. They were subjected to Single Cell Cytotoxic Assay by the same manner as in Reference Example 11-(2) to measure the cancerous cell cytotoxicity. Results are shown in Table 10.

### Table 10.

| Killer Cell | | Cytotoxicity (%) |
|---|---|---|
| Sensitized with GRA-8-H | | 21.5 |
| " | GRA-1-HA | 25.4 |
| " | GRA-2-HA | 20.3 |
| " | GRA-3-HA | 22.0 |
| " | GRA-4-HA | 18.9 |
| Control | | 10.7 |

### REFERENCE EXAMPLE 16

(1)   Peripheral blood lymphocytes obtained from a healthy adult by centrifugal separation by means of "Ficollpack" (Pharmacia Co.) were adjusted with the medium RPMI-1640 containing 10% of FCS so as to be $1.5 \times 10^6$ cells/ml. To 10 ml of it, GRA-3 was added so as to be 5, 25 or 50 ng protein/ml, and incubation was carried out at 37°C in a carbon dioxide gas incubator for 48 hours to obtain killer cells.

Using GRA-4 or Receptor 2 instead of GRA-3, killer cells were obtained likewise. Also, killer cells obtained without using any GRA (Control).

(2) After killer cells obtained in (1) above were washed twice with the medium RPMI-1640 (1,000 rpm, 10 minutes), they were adjusted with the same medium containing 10% of FCS so as to be $1.5 \times 10^6$ cells/ml, which is referred to as "Efector cells" (E). As target cells (T), those which were obtained by adjusting Daudi washed twice with the above described medium with the same medium containing 10% of FCS so as to be $1.5 \times 10^6$ cells/ml were used.

100 µl of the above described E and 100 µl of the T were blended. After the mixture was incubated at 37°C for 1 hour, a test tube containing the mixture was put in ice water to stop the reaction, and the number of survival cells was measured by carrying out dyeing with 0.2% Trypan Blue. The killer activity of E was calculated from the following formula.

Killer Activity (%) = {(Number of Cells When Using E of the Control) − (Number of Cells of Tested)}/(E' + T')

wherein E' represents the number of survival cells after 100 µl of E is incubated at 37°C for 1 hour. T' represents the number of survival cells after 100 µl of T is incubated at 37°C for 1 hour. Results are shown in Table 11.

### Table 11

| Effector Cell | Killer Activity (%) | | | |
|---|---|---|---|---|
| | Concentration (ng protein/ml) | | | |
| | 50 | 25 | 5 | Average |
| Group of GRA-3 | 10.7 | 20.4 | 29.5 | 20.2 |
| Group of GRA-4 | 18.5 | 13.1 | 2.4 | 11.3 |
| Group of Receptor-2 | 15.0 | 10.3 | 14.3 | 13.2 |

(3)     When the killer activity of killer cells induced with GRA-1 and GRA-2 by the same manner as in (1) and (2) above was measured, nearly the same activity as in the above was observed.

### REFERENCE EXAMPLE 17

(1)     GRA-1 obtained in Example 1-(1) was diluted with physiological saline to be 15 µg protein/ml, which is referred to as "Anticancer Agent No. 2".

(2)     A tumor mass of MMT was cut under asceptic conditions to obtain cubes of 5 mm. They were implanted under the derma of the back of 10 C3H/He mice (male, 7 weeks), respectively. After 7 days, growth and fixation of tumor were observed. Into 5 mice, Anticancer Agent No. 2 in (1) above was introduced by subcutaneous administration in an amount of every 300 µl per day at intervals of 2 days. The other 5 mice were used as non-treated control.

After 10 days from the first administration, tumors were removed by an operation, and average volume of them was measured. As the result, the group of control had an average volume of 142.5 $mm^3$, while the group of administration had an average volume of 18.7 $mm^3$, by which an effect of reducing tumors was confirmed.

CLAIMS

1.      A thermally denatured glycosidic linkage related antigen derived from cancerous cells, comprising a lectin receptor collected from cancerous cell membrane which can bind with a terminal galactose-and/or termainal N-acetyl-galactosamine-binding lectin, and/or a cancerous cell membrane component having a property of binding with an antibody capable of binding with the lectin receptor, each of the lectin receptor and the cancerous cell membrane component being thermally denatured, the thermally denatured glycosidic likage related antigen containing a thermally denatured protein moiety and a thermally non-denatured saccharide moiety.

2.      A process for producing a thermally denatured glycosidic linkage related antigen derived from cancer cells, comprising separating a cell membrane component from cancer cells with a termainal galactose- and/or terminal N-acetylgalactosamine-binding lectin to obtain a lectin receptor, or with an antibody capable of binding with a lectin receptor collected from cancerous cell membrane which can bind with a termianl galactose- and/or terminal N-acetylgalactosamine-binding lectin to obtain a fraction having an affinity to a terminal galactose- and/or terminal N-acetylgalactosamine-binding reagent, isolating the lectin receptor and/or the fraction to obtain a glycosidic linkage related antigen, and heating the thus obtained glycosidic linkage related antigen.

3. A component having a property of binding with an antibody which is capable of binding with a lectin receptor capable of binding with a terminal galactose- and/or terminal N-acetylgalactosamine-binding lectin.

4. A process for preparing a component having a property of binding with an antibody which is capable of binding with a lectin receptor capable of binding with a terminal galactose- and/or terminal N-acetylgalactosamine- binding lectin, which comprises treating a cancerous cell membrane component with an antibody which is capable of binding with a lectin receptor capable of binding with a terminal galactose- and/or terminal N-acetylgalactosamine- binding lectin.

5. An anticancer agent comprising a thermally denatured glycosidic linkage related antigen derived from cancerous cells. comprising a lectin receptor collected from cancerous cell membrane which can bind with a terminal galactose- and/or termainal N-acetyl-galactosamine-binding lectin, and/or a cancerous cell membrane component having a property of binding with an antibody capable of binding with the lectin receptor, each of the lectin receptor and the cancerous cell membrane component being thermally denatured, the thermally denatured glycosidic likage related antigen containing a thermally denatured protein moiety and a thermally non-denatured saccharide moiety.

FIG. 1

FIG. 2

FIG. 3

FIG. 1-A

FIG. 2-A

0106285

# FIG. 3-A

1    2    3    4

FIG. 4

FIG. 4-A

1        2              3

0106285

## FIG. 5

200K—
116K—
92.5K—
66.2K—
45K—
21K—

1   2   3   4

## FIG. 7

(−)                    (+)

## FIG. 8

# FIG. 6

TARGET CELL    ROSETTE FORMATION (%)

|  | 20 | 40 | 60 | 80 |

KATO-Ⅲ

BT-1

NOLT 4

MKN 45

Normal PBL

Normal PBL

(Y-9 plasma)

## FIG. 9

ROSETTE FORMATION (%)

100

50

0

x1000  x2000  x4000  x8000  x16000
TIME OF DILUTION (ANTIBODY-I)

## FIG. 10

ROSETTE FORMATION (%)

100

50

0    0.01        0.05   0.1            0.5        1.0
RECEPTOR-1                    (μg/tube)

## FIG. 11

92.5K—
66.2K—
45.0K—
14.4K—

1    2    3

## FIG. 12

CYTOTOXICITY (%)

30

20

10

GRA-M-3-H

CONTROL

0    100    500    1000

SENSITIZING CONCENTRATION    (ng/mℓ)

## FIG. 13